# EUROPEAN PATENT SPECIFICATION

(11) **EP 1 275 658 B1**
(45) Date of publication and mention of the grant of the patent: **15.11.2006**
(21) Application number: 01116856.4
(22) Date of filing: 10.07.2001
(51) Int. Cl.: C07K 14/015, A61K 48/00, A61K 38/16, A61K 31/70

(54) **Compositions comprising a parvovirus VP1-variant and a parvovirus NS1 protein for induction of cytolysis**
Zusammensetzung einer Parvovirus VP1-Proteinvariante und eines arvovirus NS1-Proteins zur Induktion von Zytolyse
Composé d'une variante de protéine VP1 de parvovirus et d'une protéine NS1 de parvovirus pour l'induction de cytolyse

(43) Date of publication of application: 15.01.2003
(73) Proprietor: Deutsches Krebsforschungszentrum Stiftung des öffentlichen Rechts, 69120 Heidelberg (DE)
(72) Inventor: Daeffler, Laurent, 69221 Dossenheim (DE); Nüesch, Jürg, 4132 Muttenz (CH); Rommelaere, Jean, 69118 Heidelberg (DE)
(74) Representative: Schüssler, Andrea

(56) References cited:
- WO-A-00/30668
- CAILLET-FAUQUET P. ET AL.: "Programmed killing of human cells by means of an inducible clone of parvoviral genes encoding non-structural proteins" EMBO J., vol. 9, no. 9, 1990, pages 2989-2995, XP002183642
- DUPONT F ET AL: "TUMOR-SELECTIVE GENE TRANSDUCTION AND CELL KILLING WITH AN ONCOTROPIC AUTONOMOUS PARVOVIRUS-BASED VECTOR" GENE THERAPY, MACMILLAN PRESS LTD., BASINGSTOKE, GB, vol. 7, no. 9, May 2000 (2000-05), pages 790-796, XP001000373 ISSN: 0969-7128
- KESTLER J ET AL: "CIS REQUIREMENTS FOR THE EFFICIENT PRODUCTION OF RECOMBINANT DNA VECTORS BASED ON AUTONOMOUS PARVOVIRUSES" HUMAN GENE THERAPY, XX, XX, vol. 10, 1 July 1999 (1999-07-01), pages 1619-1632, XP000943837 ISSN: 1043-0342
- MOFFATT S. ET AL.: "A cytotoxic nonstructural protein, NS1, of human parvovirus B19 induces activation of interleukin-6 gene expression" J. VIROL., vol. 70, no. 12, 1996, pages 8485-8491, XP002183643

## Description

The present invention relates to a cytotoxic pharmaceutical composition comprising DNA sequences, wherein the DNA sequences encode (a) a parvovirus NS1 protein and a parvovirus VP1 protein or (b) a parvovirus NS1 protein, a parvovirus NS2 protein and a parvovirus VP1 protein. The composition of the invention is useful as a toxin for treating tumoral diseases.

Parvovirus designates a genus of the virus family Parvoviridae. The parvovirus genus comprises a number of small, icosaedric viruses that can replicate in the absence of a helper virus. Parvovirus contains a single-stranded DNA having a length of about 5.000 bp. At the 3' and 5' ends of the DNA there is one palindromic sequence each. The DNA codes for two capsid proteins, VP1 and VP2, as well as for two regulatory non-structure proteins, NS-1 and NS-2. The latter proteins are phosphorylated and show nuclear or both cytoplasmic and nuclear localization, respectively. The two capsid proteins VP1 and VP2 are encoded by overlapping open reading frames so that the VP2 encoding region is entirely comprised within the VP1 encoding region. In a natural infection capsid proteins are expressed in a VP1:VP2 ratio of 1:10 due to alternative splicing. Recently it has been shown that the region of the capsid proteins which is specific for VP1 (N-terminal region) contains motifs common to cellular phospholipase A and indeed exerts this activity in vitro. The calcium dependent, secreted PLA₂ to which parvovirus VP1 shares similarities, takes part in signaling pathways that involve cell lysis by permeabilizing membranes. On the other hand, NS1 has been shown to be regulated by members of the protein kinase C family, which in turn are subject to regulation through phospholipases.

Parvoviruses are usually well-tolerated by populations of their natural host, in which they persist without apparent pathological signs. This is due to both the protection of foetuses and neonates by maternal immunity, and the striking restriction of parvovirus replication to a narrow range of target proliferating tissues in adult animals. This host tolerance concerns especially rodent parvoviruses, for example the minute virus of mice (MVM) and H-1 virus in their respective natural hosts, namely mice and rats. In addition, humans can be infected with the latter viruses, without any evidence of associated deleterious effects from existing epidemiological studies and clinical trials. On the other hand, autonomous parvoviruses have been shown to preferentially propagate in and to kill neoplastically transformed cells. In addition, they consist of a class of viruses that, despite causing viremia in their infected hosts, mostly produce an apathogenic infection. For these reasons, autonomous parvoviruses are thought to be excellent tools for cancer gene therapy. Particular interests are focused on recombinant vectors maintaining their natural oncotropism, as well as their oncolytic and oncosuppressive potential.

NS1, the major non-structural protein is necessary for viral DNA replication and participates in the regulation of viral gene expression. Particularly, NS1 transactivates the promoter P38 and exhibits DNA-binding, helicase and DNA-nicking activities. Furthermore, NS1 induces cytotoxic and/or cytostatic stress in sensitive host cells.

Therefore, it seems advisable to keep NS1 within parvoviral vectors, in order to (a) maintain their specific competence for NS1-dependent DNA replication and gene expression in tumour cells, resulting in an enhanced production of therapeutics agents at the desired locus (oncotropism), (b) take advantage of NS1 cytotoxicity to kill neoplastic cells and shed tumour antigens to elicit anti-cancer immunity (oncolysis), and (c) achieve still unknown effects that may contribute to the persistence (memory) of a tumour-free status (oncosuppression).

Current parvoviral vectors harbor the wild type genes (for the above discussed reasons) and either of a variety of different transgenes aimed to induce anti-tumoral responses. In most applications considered to date, the transgenes were chosen for their capacity to upmodulate the host cellular immunity against cancer. While the transgene expression induced by such vectors was successfully targeted at the location of the tumour and was especially high, duration of the therapy was relatively short, i.e. three to five days, which is likely due to the NS1-induced killing of transduced cells. New developments concerning the regulation of the NS1 protein have shown that the cytotoxicity of this protein is a regulated process. Furthermore, NS1 variants have been produced by site-directed mutagenesis of putative regulatory elements, which are up- and down-modulated regarding their cytotoxic potential while keeping replicative functions (European patent application No. 99115161.4). These variants may allow a prolongated expression of transgenes harbored by parvoviral vectors (less toxic variants) or endow infectious parvoviruses with an enhanced oncotoxicity (more toxic variants).

Considering the use of NS1 as an oncotoxin in the context of parvoviral, but also non-parvoviral vectors, it should be stated that, though able to induce apoptosis in certain cancer cells (leukemias), NS1 alone often causes only a cytostatic effect that is accompanied by morphological changes (cell shrinking) and a dramatic disorganisation of the cytoskeleton in the absence of cell lysis or apoptotic signs. NS1-expressing cells remain on a long-term in this state that has, thus, common features with a terminal differentiation. This NS1-mediated induction of a cellular terminal state is interesting on its own right for limiting tumour cell proliferation, yet it is not suitable to trigger the uptake of tumour-associated antigens (TAAs) by presenting cells of the immune system, which requires tumour cell death. This is a very serious limitation of the use of NS1 alone: Given that only a minor fraction of tumour cells can be expected to be killed, it is crucial that the direct toxicity of the vector (affecting only transduced cells) is enhanced by the immune system in order to achieve a bystander suppressive effect on non-infected tumour cells.

Therefore, it is the object of the present invention to provide a means for improving the therapeutic usefulness of NS1, e.g. as regards the NS1 cytotoxicity to kill neoplastic cells and shed tumour antigens to elicit anti-cancer immunity.

According to the invention this is achieved by the subject matters defined in the claims.

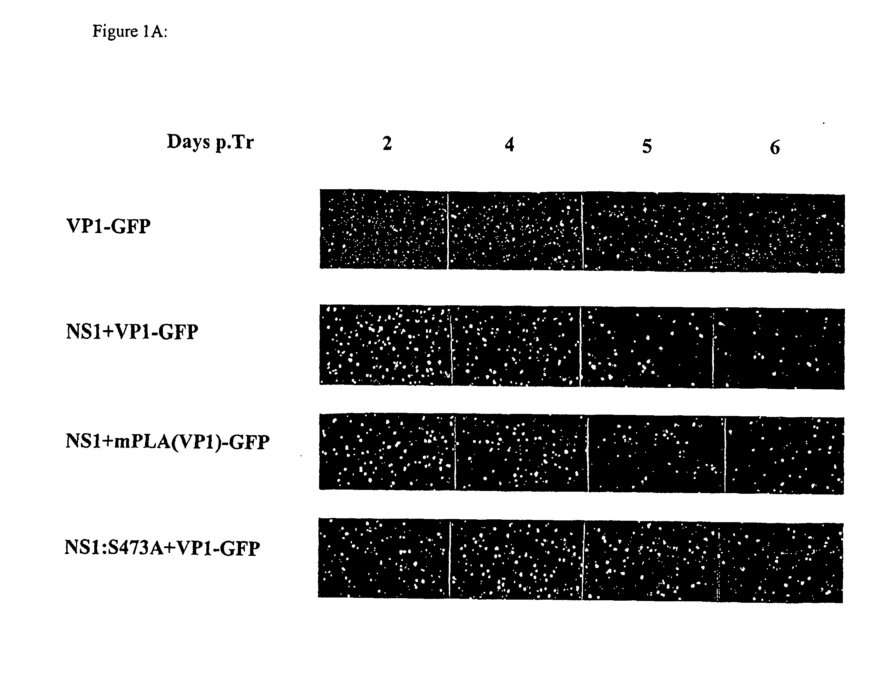

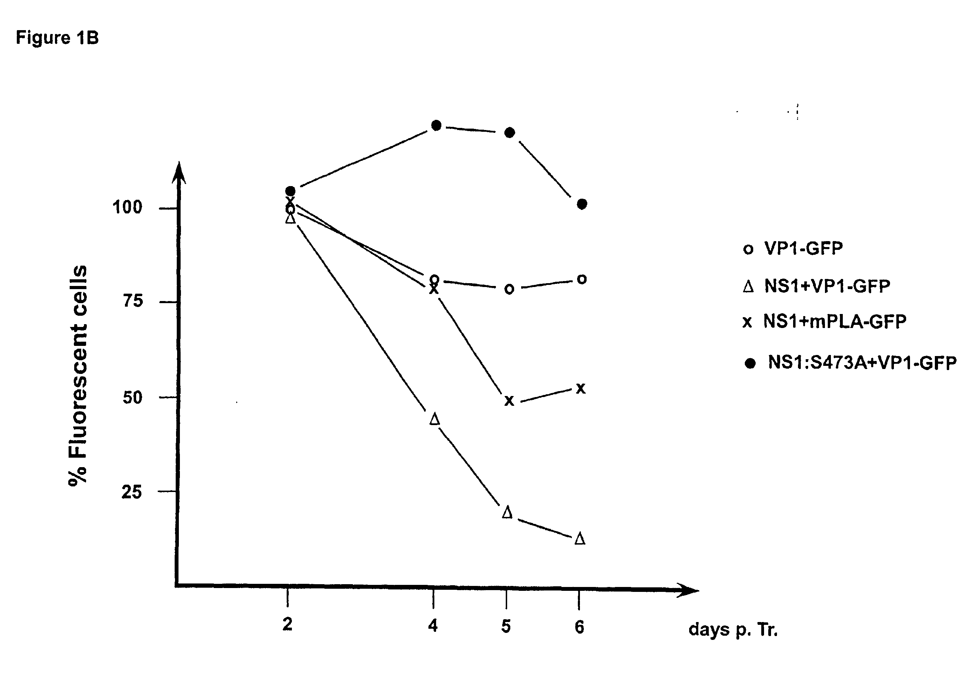

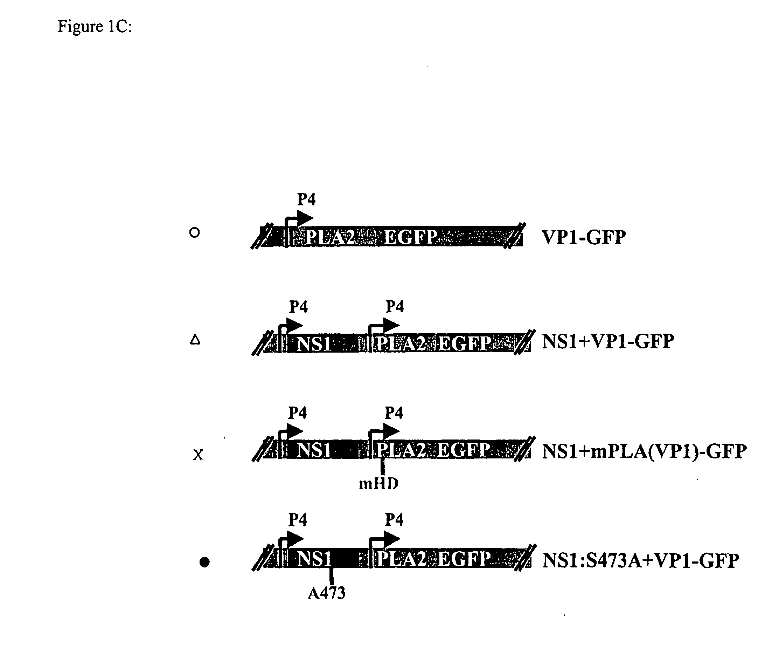

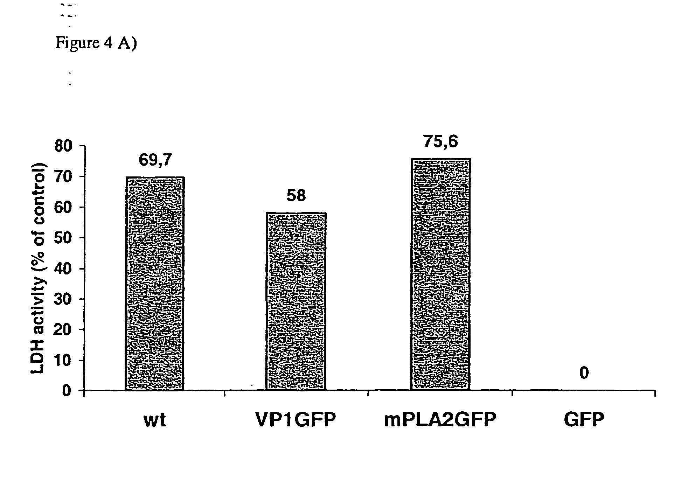

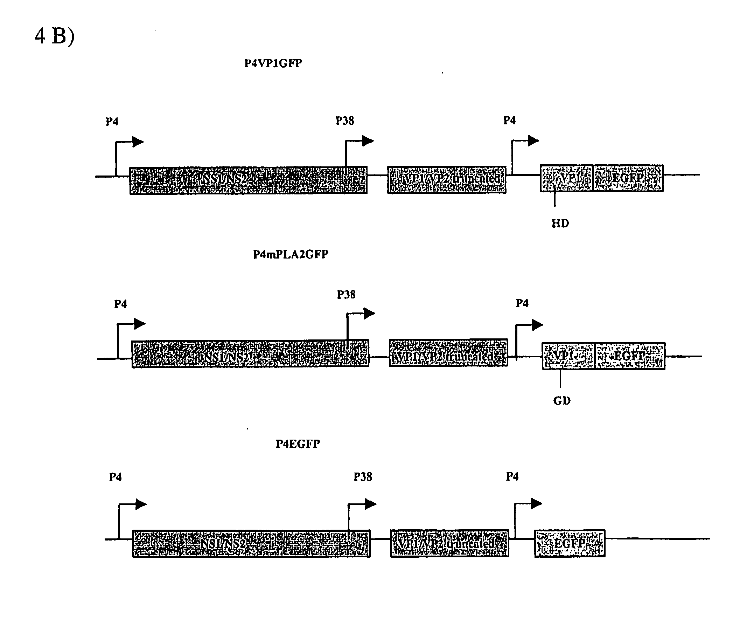

The present invention is based on the applicant's finding of a new toxin which is a combination of vectors encoding two parvoviral products, the NS1 protein and the capsid VP1 protein or a part thereof. Since VP1 is a large protein (83 kDa, 2453 bp ORF) which is itself subject to regulation (it is post translationally modified), small fusion proteins were constructed containing a VP1-specific region and expressed together with the effector protein NS1 upon transfection in mammalian cells. While being well tolerated by the cells on their own, the fusion proteins significantly increased the intrinsic cytotoxicity of NS1 as measured by the disappearance of VP1/NS1 containing cells in a function of time (monitored by fluorescent proteins present in transfected cells; see Figure 1.). This increased toxicity is manifested in an induction of cytolysis as apparent by the release of lactate dehydrogenase (LDH) in the presence of both wild type NSI and the VPI specific region, a feature that is absent upon the sole expression of wildtype NSI (Fig. 4). In this regard it has to be stated that the toxic effects of NSI and VPI could be mutual in a way that not only VPI is increasing NSI toxicity, but also that NSI is able to activate VPI induced cytolysis. To conclude, this new toxin is endowed with an enhanced capacity for causing cell death and not merely cell proliferation arrest. In the cellular system analysed, the NS1-VP1 toxin proved to have an enhanced lytic activity. Thus, tumorally expressed NS1-VP1 toxin is endowed with an enhanced capacity to kill tumour cells, which in its turn should stimulate the loading of presenting cells with TAAs and the activation of an anticancer immune response.

The present invention, provides a cytotoxic pharmaceutical composition comprising DNA sequences, wherein the DNA sequences encode (a) a parvovirus NS1 protein and a parvovirus VP1 protein or (b) a parvovirus NS1 protein, a parvovirus NS2 protein and a parvovirus VP1 protein.

The expression "parvovirus" comprises any parvovirus, particularly a rodent parvovirus, such as minute virus of mice (MVM) and H-1 virus.

The expression "NS1 protein" comprises any NS1 protein, i.e. the native protein obtainable form the parvoviruses described above as well as modified versions thereof which are biologically active (see, e.g., Genebank Accession No: PO3134; Astell et al., Nucleic Acids Res. 11 (1983), 999-1018). Such modified versions of the NS1 protein also comprise the mutants disclosed in the European patent application No. 99115161.4.

The expression "VP1 protein" comprises any VP1 protein, i.e. the native protein obtainable from the parvoviruses described above as well as modified versions and fusion proteins which are still biologically active (see, e.g., Genebank Accession No: PO3137; Astell et al., Nucleic Acids Res. 11 (1983), 999-1018). Examples of modified versions are truncated and mutant forms of the VP1 protein.

The expression "a parvovirus NS1 protein and a parvovirus VP1 protein" also comprises NS1-VP1 fusion proteins. NS1-VP1 fusion proteins comprise any NS1-VP1 fusion protein which exhibits the biological activity of both protein partners. Preferably, the NS1 protein corresponds to the N-terminal partner of the fusion protein. DNA sequences encoding such proteins can be designed by the person skilled in the art by well known methods. The person skilled in the art is also in a position to design DNA sequences encoding a fusion protein, wherein the NS1- and VP1-protein, respectively, is (are) truncated but still active.

Assays for monitoring the biological activities of the proteins (VP1; PLA₂-activity) are well known to the person skilled in the art and can be carried like measurements of arachidonic acid release (Balsinde et al., PNAS USA 95 (1998), 7951-7956) or production of prostaglandin E2 (Newton et al., 1998 , IBC 48, 32312-21) or as described in Example 2, below.

In a preferred embodiment, the VP1 protein is fused to a carrier protein allowing, e.g., to detect the expression of the VP1 protein in a host cell. Particularly preferred are the carrier proteins green fluorescent protein (GFP), NS1, IL-2, IP10, Cytatines, Cro, or signaling peptides targeting the protein to distinct locations within the cell.

In a more preferred embodiment, the VP1 protein (or the VP1 partner of the fusion protein) is a truncated protein which still exhibits the desired biological activity. Such truncated versions of the VP1 protein can be generated by the person skilled in the art according to standard methods. The biological activity of the VP1 fragments can be assayed, e.g. by the method described in Example 2, below.

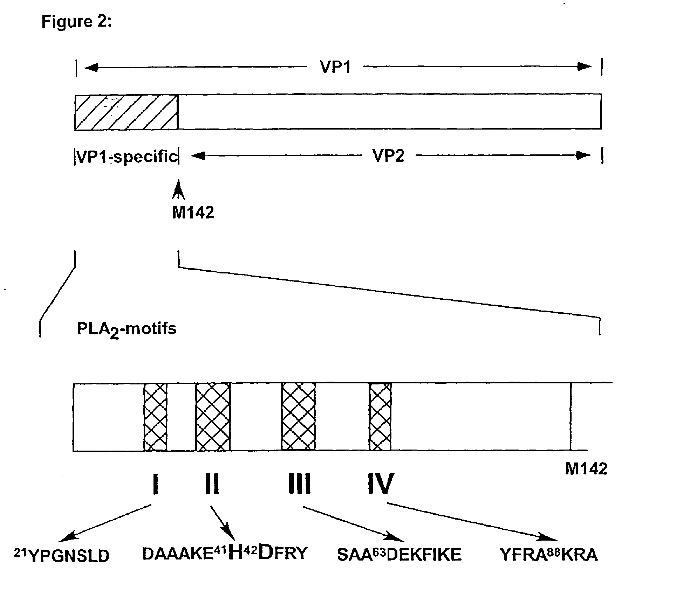

In an even more preferred embodiment, the truncated VP1 protein comprises the VP1 domain exhibiting phospholipase A activity. This activity is localised within the pecific N-terminal part of the VP1 protein (residues 1 to 142) (see Figure 2; Zadori et al. (2000), VIIIth Parvovirus Workshop Mont Tremblant, Quebec, Canada.)

A DNA sequence comprised in the composition of the present invention can be present in a vector and expression vector, respectively. A person skilled in the art is familiar with examples thereof. In the case of an expression vector for E. coli these are e.g. pGEMEX, pUC derivatives, pGEX-2T, pET3b, T7 based expression vectors and pQE-8. For the expression in yeast, e.g. pY100 and Ycpad1 have to be mentioned while e.g. pKCR, pEFBOS, cDM8, pMSCND, and pCEV4 have to be indicated for the expression in animal cells. The baculovirus expression vector pAcSGHisNT-A is especially suitable for the expression in insect cells.

In a preferred embodiment, the vector containing the DNA sequence(s) according to the invention is a virus, e.g. an adenovirus, vaccinia virus, an AAV virus or a parvovirus, such as MVM or H-1, a parvovirus being preferred. The vector may also be a retrovirus, such as MoMULV, MoMuLV, HaMuSV, MuMTV, RSV or GaLV. Suitable promoters include the strong constitutive human or mouse cytomegalovirus (CMV) immediate early promoter, the inducible P38 parvovirus promoter, the parvovirus P4 promoter and tissue and tumour specific promoters.

For constructing expression vectors which contain the DNA sequence(s) according to the invention, it is possible to use general methods known in the art. These methods include e.g. in vitro recombination techniques, synthetic methods and in vivo recombination methods as described in Sambrook et al., supra, for example. These vectors can be propagated in suitable host cells.

These host cells include bacteria, yeast, insect and animal cells, preferably mammalian cells. The E. coli strains HB101, DH1, x1776, JM101, JM109, BL21, XL1Blue and SG 13009, the yeast strain Saccharomyces cerevisiae and the animal cells L, A9, 3T3, FM3A, CHO, COS, Vero, HeLa and the insect cells sf9 are preferred. Methods of transforming these host cells, of phenotypically selecting transformants and of expressing the DNA according to the invention by using the above described vectors are known in the art.

The composition of the invention can be produced by culturing of the transformant described above under suitable conditions and isolating the protein(s) of the composition from the transformant or the medium. Methods for culturing said transformants, isolating and purifying the protein(s) are well known to the person skilled in the art.

Moreover, the pharmaceutical composition of the invention preferably comprises suitable pharmaceutical carriers. Examples of suitable pharmaceutical carriers are well known in the art and include phosphate buffered saline solutions, water, emulsions, such as oil/water emulsions, various types of wetting agents, sterile solutions etc.. Such carriers can be formulated by conventional methods and can be administered to the subject at a suitable dose.

Although this is not part of the claimed subject-matter, the present invention presupposes that administration of the composition may be effected by different ways, e.g. by intravenous, intraperetoneal, subcutaneous, intramuscular, topical or intradermal administration. The route of administration, of course, depends on the nature of the disease, e.g. tumour, and the kind of compound(s) contained in the pharmaceutical composition. The dosage regimen will be determined by the attending physician and other clinical factors. As is well known in the medical arts, dosages for any one patient depends on many factors, including the patient's size, body surface area, age, sex, the particular compound to be administered, time and route of administration, the kind of the tumour, general health and other drugs being administered concurrently. The composition of the present invention is administered in the form of (a) genetic element(s) encoding parvoviral proteins. Suitable vectors and methods for in vitro- or in vivo-gene therapy are described in the literature and are known to the persons skilled in the art; see, e.g., WO 94/29469 or WO 97/00957. The DNA sequences comprised in the composition of the present invention can be administered directly to the target site, e.g., by ballistic delivery, as a colloidal dispersion system or by catheter to a site in artery. The colloidal dispersion systems which can be used for delivery of the above DNA sequences include macromolecule complexes, nanocapsules, microspheres, beads and lipid-based systems including oil-in-water emulsions, (mixed) micelles, liposomes and lipoplexes. The preferred colloidal system is a liposome. The composition of the liposome is usually a combination of phospholipids and steroids, especially cholesterol. The skilled person is in a position to select such liposomes which are suitable for the delivery of the desired DNA molecule. Organ-specific or cell-specific liposomes can be used, e.g. in order to achieve delivery only to the desired tissue. The targeting of liposomes can be carried out by the person skilled in the art by applying commonly known methods. This targeting includes passive targeting (utilising the natural tendency of the liposomes to distribute to cells of the RES in organs which contain sinusoidal capillaries) or active targeting (for example by coupling the liposome to a specific ligand, e.g., an antibody, a receptor, sugar, glycolipid, protein etc., by well known methods). In the present invention monoclonal antibodies are preferably used to target liposomes to specific tumours via specific cell-surface ligands.

Although treatment in vivo of the animal or human body is excluded from the scope of the invention, the invention presupposes that treatment can be given to cell cultures or organisms, e.g. tumour bearing organisms. Should the DNAs in the composition of the present invention be administered as separated genetic elements, time intervals between their respective administration can be applied. Alternatively, stably transfected cell lines expressing the VP1 protein can be produced. In the absence of NS1, the VP1 component is well tolerated by host cells over extended periods of time. The cytolytic effect is exerted only when the VP1 component is coexpressed with the NS1 component. It is, thus, possible to select tumour cell lines that stably express the VP1 component and contain (or not) an inducible clone of the NSI component that is kept silent. Upon NS1 expression (as a result of NS1 gene transfer or induction) the tumour cells get lyzed, which can be achieved in vitro or in vivo (after transplantation of VP1 expressing cells) in order to trigger the release, uptake and presentation of tumour-associated antigens. Moreover, the composition of the invention or the corresponding DNA sequences can be applied in combination with other agents (such as cytokines) in gene and cancer therapy approaches.

Finally, the composition of the present invention is useful for the preparation of a toxin for treating tumoral diseases.

### Brief description of the drawings

Figure 1: A9 cells grown on Spot-Slides (NeoLab, Heidelberg, Deutschland) were transfected with the indicated constructs (100 ng/well) using 1 µL Lipofectamine (GibcoBRL, Karlsruhe, Deutschland). Transfected, living cells were identified by their green fluorescence and monitored in daily intervals using a reverse angle fluorescence Microscope (Leica IL2).
   **(A)** Photographs of fluorescent, transfected cells obtained after 2, 4, 5 and 6 days post transfection.
   **(B)** Statistical evaluation of NS1/VP1 toxicity. Transfected, GFP expressing (fluorescent) cells were counted at daily intervals and their survival was expressed as percentage of the number of fluorescent cells at day 2 post transfection.
      Schematic presentation of the effector constructs expressing PLA₂ (the active domain of the VP1 polypeptide) and/or NS1 parvovirus MVM. Both effector genes are expressed under the control of the parvoviral P4 promoter. To monitor cells containing transfected DNA, the gene for the green fluorescent protein was fused to the PLA₂ domain. The phosphorylation site mutant NS1:S473A (Dettwiler et al., J.Virol. 73 (1999), 7410-7420) was chosen as a representative non-toxic NS1 mutant.
      Mutant PLA₂ was generated by PCR replacing the HD-motif (aa 41/42), i.e. the active site of the PLA₂ enzyme, by methionine residues. Toxicity assay was carried out according to Corbau et al., Virology 278 (2000), 151-167.
      As shown in (A) and (B) , wild type NS1 and the PLA₂-domain of VP1 acted synergistically in that this cytotoxicity of coexpressed active polypeptides was in comparison with the effect of each effector protein alone, as revealed by the disappearance of fluorescent/transfected cells.
Figure 2: Schematic representation of the parvovirus VP proteins
   **(A)** VP1, the large 83 kDa capsid protein comprises 716 amino acids while VP2 lacks the 142 N-terminal amino acids and starts at methionine 143 due to alternative splicing of the precursor mRNAs. The VP1 unique region is represented as a hatched box.
   (**B**) Blow-up of the VP1-specific region comprising amino acids 1 to 142 of the VP1 protein. The four motifs showing homology to cellular PLA₂s are indicated as crossed regions and the corresponding amino acid sequences of VP1 are given on the bottom. The putative active site for PLA₂-activity is indicated by capitals.

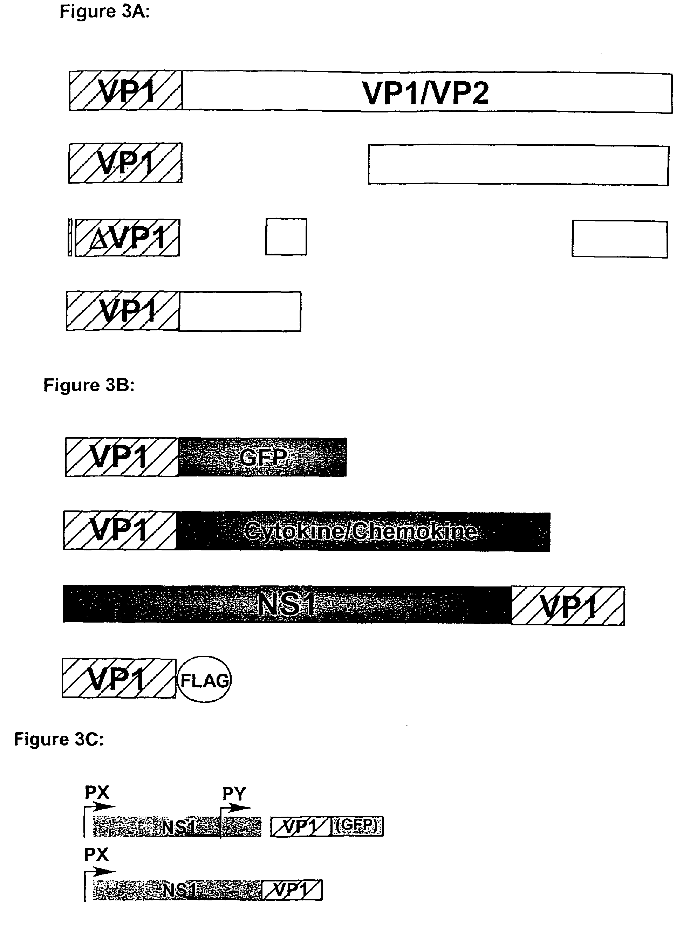

Figure 3: Schematic representation of examples of VP1 and VP1 variants designed for use of the present invention
   **(A)** VP1 deletion mutants of various sizes
   **(B)** VP1 fusion peptids using "carrier"-polypeptides

   Examples of cassettes for the expression of VP1-variants to obtain synergistic effects together with NS1
Figure 4 The experiment involves the use of the Promega kit "CytoTox 96®" non-radioactive cytotoxicity assay (Promega, Mannheim, Deutschland). LDH is a cytosolic enzyme that is released upon cell lysis. Released LDH is measured using an enzymatic assay which results in the conversion of a tetrazolium salt (INT) into a red formazan product. The colour intensity, measured with a standard 96 well plate reader at 490 nm, is proportional to the number of lysed cells. Experiments were performed in 96 well plates containing 2000 mouse A9 cells per well. Infection was carried out 24 hours after spreading of the cells at a multiplicity of 10 infectious units of virus per cell. Culture medium was collected 6 days after transfection and directly tested for LDH activity as described by the manufacturer. The total amount of LDH in the cells stands for 100%
   (B) Schematic representation of VP1 constructs used for the LDH assay. Their construction is detailed in Example 1C.

The present invention is explained by the examples.

### Example 1

### Construction of vectors for expression of VP1 variants

### (a) pP4-VP1-GFP

The coding sequence for the VP1-GFP fusion protein was obtained by chimeric polymerase chain reaction (PCR) using the primer pair 5'-ATG ACT CCA TGG CGC CTC CAG CTA AAA AGA-3' (A) and 5'-TCG CCC TTG CTC ACC ATC GTT TGA CTC CTT TGC TG-3' (B) using as template pTM1-VP1 to obtain the coding sequence for the VP1-specific domain of parvovirus VP-proteins. The primer pair 5'-AGG AGT CAA ACG ATG GTG AGC AAG GGC GAG GAG-3' (C) and 5'-ACG GTC TCG AGT AGC GAC CGG CGC TCA GTT GG-3' (D) with pEGFP (Clontech, Heidelberg, Deutschland) as a template was used to obtain the GFP encoding sequence. In a second PCR reaction the VP1- and GFP-fragments were annealed due to overlapping sequences and further amplified using primers A and D. After amplification the PCR product was digested with the restriction endonucleases NcoI and XbaI and ligated into the similarly cleaved vector pDB-MVMp; see Figure 1C. pDB-MVMp constitutes an infectious molecular clone of MVMp (Kestler et al., Hum. Gene Ther. 10 (1999), 1619-1632).

### (b) pP4-NS1-VP1

The construct constitutes an eukaryotic expression vector under the control of the parvovirus P4 promoter (S-phase/transformation regulated). The coding sequence for the NS1-VP1 fusion protein was obtained by chimeric PCR using the primer pair 5'-GGT CAA TCT ATT CGC ATT GAT-3' (A) and 5'-GAG GCG CCA TGT CCA AGT TCA GCG GCT CGC-3' (B) for amplification of the template pTM1-NS1 Nüesch et al., Virology 191 (1992), 406-416) to obtain the full-length NS1 fragment. The primer pair 5'-GAA CTT GGA CAT GGC GCC TCC AGC TAA AAG-3' (C) and 5'-ATA CGC CCG GGC TAG GTT TGA CTG CTT TGC TGT-3' (D) with pTM1-VP1 as a template was used for obtaining the VP1 coding sequence. In a second round of PCR the two fragments were combined using primers A and D. After amplification the PCR product was digested with the restriction endonucleases XhoI and SmaI and ligated into XhoI and StuI digested vector pTHis-NS1 (Nüesch et al., Virology 209 (1995), 122-135). To obtain pP4-NS1-VP1, the coding sequence of the NS1-VP1 variant was amplified by PCR using an N-terminal primer containing a SmaI restriction site together with a C-terminal primer containing an XbaI cleavage site. This SmaI to XbaI NS1-VP1 fragment wad then transferred into EcoRV and XbaI cleaved pP4-GFP. Clone pP4-NS1-VP1 was deposited on May 15, 2001, with the DSMZ under accession number DSM 14300 according to the regulations of the Budapest treaty.

### (c) pP4-NS1+2-P4-VP1-GFP

To obtain pP4-NS1-P4-VP1-GFP used for transfection experiments, the PmeI to XbaI fragment of pP4-VP1-GFP (or for controls pP4-GFP, pP4-mPLA-GFP) was transferred into the StuI and XbaI cleaved pDB-MVMp or pDB-NS1:S473A (Corbau et al., Virology 278 (2000), 151-167).

To obtain pP4-NS1+2-P4-VP1-GFP recombinant MVM virus; the P4-VP1-GFP fragment was amplified using a primer 5'-ATA CGA GAT CTG TTT AAA CCA AGG CGC GAA AAG G-3' within the P4 promoter region containing an N-terminal BglII cleavage site and primer D wihtin the C-terminus of GFP harbouring an XbaI site. This BglII to XbaI fragment was then cloned into the BglII and XbaI cleaved capsid region of pDB-MVMp. Clone pP4-NS1+2-P4-VP1-GFP was deposited on May 15, 2001, with the DSMZ under accession number DSM 14301 according to the regulations of the Budapest treaty.

Similarly, the BglII to XbaI fragments of P4-GFP and P4-mPLA-GFP were transferred into the infectious DNA clone of MVMp.

### Example 2:Toxicity assays

### (a)

A9 cells grown on Spot slides (Neolab) were transfected with the constructs described in Example 1, above, and shown in Figures 1C and 3 expressing the GFP (fusion) proteins using lipofectamine. Fluorescent cells were monitored in daily intervals for their overall structure. From the results shown in Figure 1B it can be concluded that NS1 and VP1 fusion proteins act synergistically to kill the transfected A9 cells. Experimental details are given in the legend of Figure 1.

### (b)

A9 cells were transduced with various multiplicities of recombinant parvoviruses containing VP1-variants or mutants thereof. In daily intervals the release of endogenous LDH (lactate dehydrogenase) was measured using commercial kits in order to obtain measurements for cytolysis. Experimental details are given in the legend of Figure 4.

## Claims

1. A cytotoxic pharmaceutical composition comprising DNA sequences, wherein the DNA sequences encode (a) a parvovirus NS1 protein and a parvovirus VP1 protein, or (b) a parvovirus NS1 protein, a parvovirus NS2 protein and a parvovirus VP1 protein.

2. The composition of claim 1(a), wherein the VP1 protein is fused to a carrier protein.

3. The composition of claim 2, wherein the carrier protein is green fluorescent protein (GFP).

4. The composition of any one of claims 1 to 3, wherein the VP1 protein is a truncated VP1 protein.

5. The composition of claim 4, wherein the truncated VP1 protein comprises the VP1 domain exhibiting phospholipase A activity.

6. The composition of anyone of claims 1 to 5, wherein the DNA sequences are inserted into an expression vector.

7. The composition of claim 6, wherein said expression vector is a parvovirus vector.

8. The composition of claim 7, wherein said expression vector is pP4-NS1-VP1 (accession number DSM 14300) or pP4-NS1+2-P4-VPl-GFP (accession number DSM 14301)

## Patentansprüche

1. Zytotoxische pharmazeutische Zusammensetzung umfassend DNA Sequenzen, wobei die DNA Sequenzen (a) ein Parvovirus NS1 Protein und ein Parvovirus VP1 Protein, oder (b) ein Parvovirus NS1 Protein, ein Parvovirus NS2 Protein und ein Parvovirus VP1 Protein kodieren.

2. Zusammensetzung nach Anspruch 1 (a), wobei das VP 1 Protein mit einem Trägerprotein fusioniert ist.

3. Zusammensetzung nach Anspruch 2, wobei das Trägerprotein grün-fluoreszierendes Protein (GFP) ist.

4. Zusammensetzung nach einem der Ansprüche 1 bis 3, wobei das VP1 Protein ein trunkiertes VP1 Protein ist.

5. Zusammensetzung nach Anspruch 4, wobei das trunkierte VP1 Protein die VP1 Domäne umfasst, die Phospholipase A Aktivität zeigt.

6. Zusammensetzung nach einem der Ansprüche 1 bis 5, wobei die DNA Sequenzen in einen Expressionsvektor eingefügt sind.

7. Zusammensetzung nach Anspruch 6, wobei der Expressionsvektor ein Parvovirus Vektor ist.

8. Zusammensetzung nach Anspruch 7, wobei der Expressionsvektor pP4-NS1-VP1 (Hinterlegungsnummer DSM 14300) oder pP4-NS1+2-P4-VP1-GFP (Hinterlegungsnummer DSM 14301) ist.

## Revendications

1. Composition pharmaceutique cytotoxique comprenant des séquences ADN, **caractérisée en ce que** les séquences ADN encodent (a) une protéine parvovirus NS1 et une protéine parvovirus VP1, ou (b) une protéine parvovirus NS1, une protéine parvovirus NS2 et une protéine parvovirus VP1.

2. Composition selon la revendication 1, dans laquelle la protéine VP1 est fusionnée avec une protéine porteuse.

3. Composition selon la revendication 2, dans laquelle la protéine porteuse est une protéine verte fluorescente (GFP).

4. Composition selon l'une quelconque des revendications 1 à 3, dans laquelle la protéine VP1 est une protéine VP1 tronquée.

5. Composition selon la revendication 4, dans laquelle la protéine VP1 tronquée comprend le domaine VP1 ayant une activité phospholipase A.

6. Composition selon l'une quelconque des revendications 1 à 5, dans laquelle les séquences ADN sont insérées dans un vecteur d'expression.

7. Composition selon la revendication 6, dans laquelle les séquences ADN sont insérées dans un vecteur parvovirus.

8. Composition selon la revendication 7, dans laquelle le vecteur d'expression est pP4-NS1-VP1 (numéro d'accès DSM 14300) ou bien pP4-NS1+2-P4-VP1-GFP (numéro d'accès DSM 14301).
